# EUROPEAN PATENT APPLICATION

(11) **EP 4 714 425 A1**
(43) Date of publication of application: **25.03.2026**
(21) Application number: 24893138.8
(22) Date of filing: 23.10.2024
(51) Int. Cl.: A61K 8/362, A61K 8/34, A61Q 19/00, A61Q 5/02, A61P 17/00, C08G 83/00

(54) **AZELAIC ACID/BETAINE/PANTHENOL SELF-RECOGNITION DES SYSTEM AND PREPARATION METHOD**

(30) Priority: 20.11.2023 CN 202311543929
(71) Applicant: Wuxi Zhiyan Biotechnology Co., Ltd., Wuxi, Jiangsu 214194 (CN)
(72) Inventor: CAI, Beilei, Wuxi, Jiangsu 214194 (CN); TIAN, Zhihui, Wuxi, Jiangsu 214194 (CN); WANG, Yue, Wuxi, Jiangsu 214194 (CN); QIAN, Xin, Wuxi, Jiangsu 214194 (CN); YANG, Shuyan, Wuxi, Jiangsu 214194 (CN); DU, Liyong, Wuxi, Jiangsu 214194 (CN); YANG, Jingguo, Wuxi, Jiangsu 214194 (CN)
(74) Representative: Santarelli
(86) International application number: PCT/CN2024/126713
(87) International publication number: WO 2025/107979

(57) **Abstract**

Disclosed in the present invention are an azelaic acid/betaine/panthenol self-recognition DES system and a preparation method, belonging to the technical field of supermolecules. The preparation method for the azelaic acid/betaine/panthenol self-recognition DES system of the present invention comprises the following steps: mixing azelaic acid, betaine, and panthenol according to a molar ratio of 1:0.4-6:0.7-10, and stirring and heating to obtain an azelaic acid/betaine/panthenol self-recognition DES system. The azelaic acid/betaine/panthenol self-recognition DES system prepared according to the present invention is readily soluble in water, and can be directly diluted by adding water until the mass concentration of azelaic acid is 0.8%-35%, without phase separation and solid precipitation, thereby effectively improving the water solubility and stability of azelaic acid, with the degree of improvement far exceeding that of the prior art.

## Description

### Technical Field

The present invention relates to an azelaic acid/betaine/panthenol self-recognition DES system and a preparation method, belonging to the technical field of supermolecules.

### Background of the Invention

Azelaic acid demonstrates excellent efficacy in prophylaxis and treatment of skin symptoms such as acne, lentigo maligna, chloasma, melasma, malignant melanoma, and pigmentation. However, azelaic acid exhibits poor solubility in both water and oil, has a relatively high melting point, and is prone to precipitation at a high addition amount, which results in system roughness and instability. Moreover, single dosage forms make it difficult to prepare aqueous and refreshing products suitable for large-area application on oily skin.

In the prior art, solubility problems of azelaic acid are solved through methods such as ultrafine grinding (CN 114181072 A), supramolecular complex formation by compounding organic solvents with azelaic acid (CN 110669226 A), cocrystallization of azelaic acid with organic substances (CN 112624918 A), encapsulation treatment of azelaic acid (CN 108187070 A), and solubilization, plasticization, and nucleation rate reduction (CN113520890A). However, the ultrafine grinding process additionally increases the cost of azelaic acid as a raw material and fails to achieve significant solubilization of azelaic acid. Compounding an organic solvent with azelaic acid to form a supramolecular complex results in limited solubility of azelaic acid, with an improvement only by 3.3 times. The cocrystallization of azelaic acid with organic substances requires a series of subsequent operations such as membrane filtration, recrystallization, and organic solvent removal to obtain target cocrystals, which is environmentally unfriendly and complex. Although the encapsulation treatment of azelaic acid enhances the solubility of azelaic acid to a certain extent, relatively complex process flow and required use of massive cyclodextrin results in that an inclusion system is prone to stickiness during use and causes a poor skin feeling. The solubilization, plasticization, and nucleation rate reduction require the addition of a large amount of stabilizers to form colloids, which inevitably hinders the penetration of azelaic acid into the skin.

Therefore, there is an urgent need to solve the problems of water solubility and stability of azelaic acid through a simple and green processing technique.

### Summary of the Invention

### [Technical Problems]

Currently, methods for enhancing the water solubility of azelaic acid have defects such as operation complexity, extensive use of organic solvents, limited solubility improvement, and impact on later use.

### [Technical Solution]

To overcome the above defects, the present invention selects betaine and panthenol as compatible molecules, and prepares a liquid supramolecular DES system through DES modification technology and azelaic acid self-recognition, which enhances the solubility and stability of azelaic acid, endows a system with excellent moisturizing performance, expands an application scope of azelaic acid, and enables azelaic acid to be better integrated into water-based cosmetic formulations.

A first objective of the present invention is to provide a method for preparing an azelaic acid/betaine/panthenol self-recognition DES system, and the method includes the following steps:
mixing azelaic acid, betaine, and panthenol according to a molar ratio of 1:0.4-6:0.7-10, and stirring and heating to obtain an azelaic acid/betaine/panthenol self-recognition DES system.

As an embodiment of the present invention, an atmosphere for stirring and heating is natural air.

As an embodiment of the present invention, the stirring and heating refers to stirring and heating at 350-850 rpm and 50-115°C for 1-15 hours; and further preferably, the stirring and heating is performed at 350-850 rpm and 60-115°C for 1-15 hours.

As an embodiment of the present invention, cooling to room temperature is required after stirring and heating.

According to a second objective of the present invention, the azelaic acid/betaine/panthenol self-recognition DES system prepared by the method of the present invention is in a liquid state.

As an embodiment of the present invention, maximum content of azelaic acid in the azelaic acid/betaine/panthenol self-recognition DES system reaches up to 50 wt%.

As an embodiment of the present invention, the azelaic acid/betaine/panthenol self-recognition DES system remains stable without solid precipitation for at least 24 hours.

As an embodiment of the present invention, the azelaic acid/betaine/panthenol self-recognition DES system is readily soluble in water, and can be directly diluted by adding water until a mass concentration of azelaic acid is 0.8%-35%, without phase separation or solid precipitation, that is, the DES system remains intact.

As an embodiment of the present invention, the azelaic acid/betaine/panthenol self-recognition DES system has an absorption peak in a waveband range of 3,500-3,200 cm⁻¹ corresponding to an absorption peak of hydroxyl groups (hydrogen bonds); and there are peaks at 1,650-1,700 cm⁻¹ and 1,710 cm⁻¹.

A third objective of the present invention concerns an application of the azelaic acid/betaine/panthenol self-recognition DES system of the present invention in the field of daily chemicals or drugs.

As an embodiment of the present invention, the daily chemicals include cosmetics, shampoos, mouthwashes, and the like.

A fourth objective of the present invention is to provide a cosmetic product containing the azelaic acid/betaine/panthenol self-recognition DES system of the present invention.

As an embodiment of the present invention, an addition amount of the azelaic acid/betaine/panthenol self-recognition DES system accounts for 0.1-70 wt%.

As an embodiment of the present invention, the cosmetic product is of an aqueous type, a refreshing type, or the like.

As an embodiment of the present invention, the cosmetic product includes an essence serum, essence lotion, spray, skincare foam, emulsion, shampoo, and the like.

As an embodiment of the present invention, formula components in percentage by mass include:
glycerol (2-4%), butylene glycol (2-4%), phenoxyethanol (0.3-0.5%), caprylyl glycol (0.3-0.5%), a *Sphingomonas* ferment extract (0.05-0.15%), sodium hyaluronate (0.04-0.06%), polyacryloyldimethyl taurate (0.05-0.15%), an azelaic acid/betaine/panthenol self-recognition DES system (10-30%), a white willow bark extract (1-3%), disodium ethylenediaminetetraacetate (EDTA) (0.04-0.06%), a portulaca oleracea extract (1-3%), and a balance of water.

As an embodiment of the present invention, a method for preparing an essence includes:
adding water, glycerol, butylene glycol, phenoxyethanol, caprylyl glycol, and disodium EDTA into a main vessel, dissolving uniformly, and heating to 75°C;
adding sodium hyaluronate, a *Sphingomonas* ferment extract, and polyacryloyldimethyl taurate into the main vessel, and stirring uniformly; and
cooling to 45°C, adding an azelaic acid/betaine/panthenol self-recognition DES system, a white willow bark extract, and a portulaca oleracea extract, and stirring uniformly to complete the preparation.

A fifth objective of the present invention is to provide a drug for removing pigmentation and acne that contains the azelaic acid/betaine/panthenol self-recognition DES system of the present invention.

As an embodiment of the present invention, an addition amount of the azelaic acid/betaine/panthenol self-recognition DES system accounts for 0.1-70 wt%.

As an embodiment of the present invention, the drug for removing pigmentation and acne is one of a gel agent, spray, liquid preparation, and cream.

As an embodiment of the present invention, formula components in percentage by mass include:
the azelaic acid/betaine/panthenol self-recognition DES system (10-30%), docusate sodium (0.03-0.06%), disodium EDTA (0.05-0.15%), glycerol (3-5%), poloxamer 124 (0.1-0.3%), propylene glycol (3-5%), SIMULGEL 600 PHA (acrylamide/sodium acryloyldimethyl taurate copolymer & isohexadecane & polysorbate 80 & sorbitan oleate) (3-5%), and a balance of water.

As an embodiment of the present invention, a method for preparing a gel agent drug is as follows:
(1) Preparation of an active phase:
   adding poloxamer 124 to water and stirring uniformly to obtain a solution, further stirring until the solution is dissolved and becomes clear, and then adding the azelaic acid/betaine/panthenol self-recognition DES system and homogenizing with a homogenizer at 10,000 rpm for 30 minutes for later use;
(2) preparation of an aqueous phase:
   adding propylene glycol and glycerol to water to obtain a solution, dissolving the solution until it becomes clear, and then adding and stirring docusate sodium at 1000 rpm to further form a clear solution for later use;
(3) mixing:
   sequentially adding the aqueous phase and the active phase to a kettle, rinsing a beaker containing the active phase with water, and then adding SIMULGEL 600 PHA, homogenizing and stirring at 2,800 rpm for 45 minutes;
(4) filling:
   manually filling a resulting mixture into an empty plastic tube to obtain a gel agent.

A sixth objective of the present invention is to provide a method for synergistically improving a moisturizing effect of azelaic acid by using betaine and panthenol, and the method includes the following steps:
mixing azelaic acid, betaine, and panthenol according to a molar ratio of 1:0.4-6:0.7-10, and stirring and heating to obtain an azelaic acid/betaine/panthenol self-recognition DES system with good moisturizing performance.

A seventh objective of the present invention is to provide a method for enhancing water solubility and stability of azelaic acid by using betaine and panthenol, and the method includes the following steps:
mixing azelaic acid, betaine, and panthenol according to a molar ratio of 1:0.4-6:0.7-10, and stirring and heating to obtain an azelaic acid/betaine/panthenol self-recognition DES system with good water solubility and stability.

### [Beneficial Effects]

(1) For the DES modification technology, screening of compatible molecules matching target molecules is a key challenge. In the present invention, suitable compatible molecules (betaine and panthenol) enables each molecule in the DES modification technology to self-recognize and form a supramolecular organism according to a specific molar ratio (1:0.4:0.7-1:6:10), thereby resolving drawbacks in application of the target molecules (such as azelaic acid) such as poor solubility, low bioavailability, low safety, and poor stability. Moreover, suitable compatible molecules (such as betaine and panthenol) also play a synergistic effect (such as the moisturizing effect), which enhances the efficacy of the entire DES system, and further broadens application prospects.
(2) **The** present invention changes a physical state and solubility of azelaic acid through formation of supramolecular hydrogen bonds between molecules via DES modification technology, and prepares a transparent azelaic acid/betaine/panthenol self-recognition DES system with maximum azelaic acid content of 50 wt%, which greatly enriches application scenarios of azelaic acid.
(3) **The** azelaic acid/betaine/panthenol self-recognition DES system prepared according to the present invention is readily soluble in water, and can be directly diluted by adding water until the mass concentration of azelaic acid is 0.8%-35%, without phase separation and solid precipitation, thereby effectively improving the water solubility and stability of azelaic acid, with the degree of improvement far exceeding that of the prior art.
(4) Betaine and panthenol are selected as compatible components for the azelaic acid/betaine/panthenol self-recognition DES system prepared by the present invention, which greatly enhances the moisturizing efficacy of the system.
(5) A preparation process of the azelaic acid/betaine/panthenol self-recognition DES system in the present invention is simple, safe, and pollution-free, and additional pretreatment or post-treatment of an obtained ternary liquid DES system is not required, which is suitable for large-scale promotion due to direct use as a raw material in the fields of drugs or daily chemicals.

### Brief Description of the Drawings

FIG. 1 is an infrared spectrogram of an azelaic acid/betaine/panthenol self-recognition DES system of Example 1.
FIG. 2 illustrates a thermogravimetric analysis curve of the azelaic acid/betaine/panthenol self-recognition DES system of Example 1.
FIG. 3 is a moisturizing performance evaluation chart of the azelaic acid/betaine/panthenol self-recognition DES system of Example 1.
FIG. 4 is a moisturizing performance evaluation chart of a liquid DES system product of Comparative Example 2.

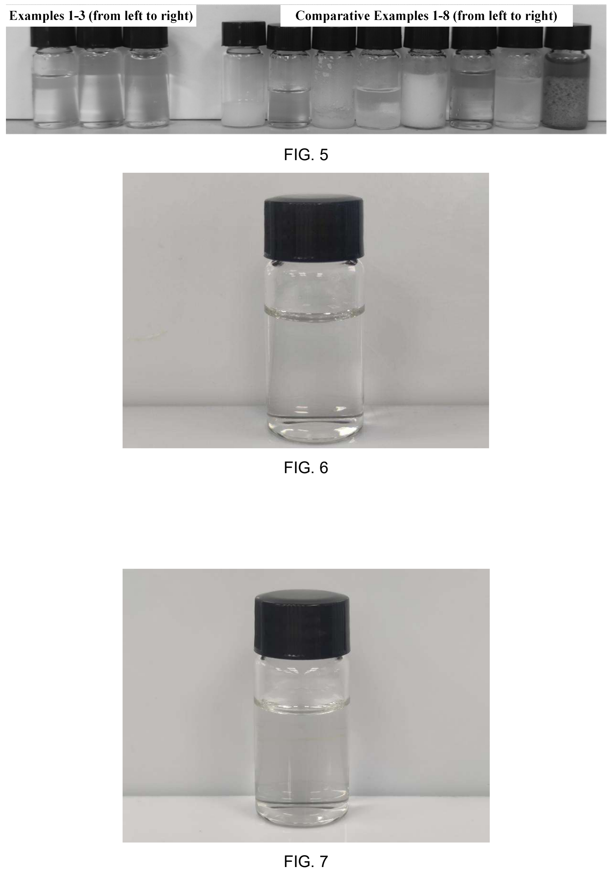

FIG. 5 is a characteristic diagram of samples prepared in Examples 1-3 and Comparative Examples 1-8 after storage for 24 hours.
FIG. 6 is a characteristic diagram of a sample prepared in Example 4 after storage for 24 hours.
FIG. 7 is a characteristic diagram of a sample prepared in Example 5 after storage for 24 hours.

### Detailed Description of Embodiments

Preferred embodiments of the present invention are described below, and it should be understood that the embodiments are intended to better explain the present invention and are not intended to limit the present invention.

### Test methods:

1. Fourier-transform infrared spectroscopy (FTIR) test:
   Machine specification: FTS6000; machine manufacturer: Bio-Rad Laboratories, USA; and experimental parameters: a scanning range of 500-4000 cm⁻¹, and a resolution of 4 cm⁻¹.
2. Thermogravimetric analysis (TGA) test:
   Machine specification: Mettler TGA2 SF/1100; machine manufacturer: Mettler Toledo, Switzerland; and experimental parameters: a temperature range of 25-450°C, a heating rate of 10°C/minute, and an experimental atmosphere of nitrogen with a flow rate of 50 mL/minute.
3. Moisturizing performance test:
   A ternary liquid deep eutectic solvent (DES) system is diluted by adding water until an azelaic acid mass fraction of 4% for moisturizing performance evaluation;
   a capacitance method is used to measure moisture content of stratum corneum of human skin, and its principle is based on significant differences in dielectric constants between water and other substances. A measured capacitance value of the skin varies due to differences in skin moisture content, and an observed parameter represents a skin moisture value;
   environmental conditions of test: a test environment temperature is 20-25°C, a humidity is 40%-60% RH, and real-time dynamic monitoring is performed;
   volunteer requirements: 40 eligible volunteers, aged 20 to 50, excluding pregnant or lactating women, where a baseline capacitance value measured by a capacitance-based skin moisture meter in a forearm test area is 15-45; no severe systemic diseases, immunodeficiency or autoimmune diseases; no active allergic diseases; no history of severe allergies to skincare cosmetics; no experience of using hormonal drugs and immunosuppressants within the past month; no experience of participating in any other clinical trials; having an experience of using test substances as prescribed and having complete data; all the volunteers should sign an informed consent form before the test; and
   preparation before the test: no experience of using any products (cosmetics or topical medications) for test sites three days before the test. Before the test, subjects need to agree to clean inner sides of forearms of both hands, and dry them thoroughly with paper towels. After cleaning, measurement areas are marked on the inner sides of forearms of both hands of the subjects. Before the official test, the subjects should sit quietly in a standard room for at least 30 minutes, during which they cannot drink water, but should expose their forearms in a test state, and keep relaxed.
   Data test: First, a blank value of a test area (data at T0) is measured, then a test substance is uniformly applied to the test area at a dosage of 2.0±0.1 mg/cm² by using latex finger cots, and skin moisture content of the test area is measured at 2 hours, 4 hours, and 5 hours after applying respectively. Each of eight subjects in each group receives five repeated tests each time, and measurement values are averaged.
4. High-low temperature cycle test:
   Cycle storage is performed at -5°C and 40°C for 15 times; and specifically, a supramolecular self-assembly system is frozen at -5°C and then dissolved at 40°C to complete one cycle, then the cycle is repeated, and a state (such as a stable and homogeneous liquid state without solid precipitation) of the supramolecular self-assembly system is observed after dissolution each time.

### Raw materials used in the examples:

Azelaic acid: commercially available, having a particle size of 500 nm-100 µm, without impact from the particle size, where the particle size selected in examples and comparative examples is 30 µm;
panthenol: D-panthenol or DL-panthenol, both of which may be used in examples and comparative examples, commercially available;
a *Sphingomonas* ferment extract: purchased from Shanghai Youshi Industrial Co., Ltd.;
polyacryloyldimethyl taurate: 99%, purchased from Wuhan Lvjing Fenghua Biotechnology Co., Ltd.;
a white willow bark extract: purchased from Plamed Green Science Group;
disodium ethylenediaminetetraacetate (EDTA): 99%, purchased from Shanghai Yuanye Bio-Technology Co., Ltd.;
a Portulaca oleracea extract: purchased from Shanghai Huiwen Biology; and
the "%" mentioned in examples and comparative examples refers to a mass percentage unless otherwise specified.

### Example 1

A method for preparing an azelaic acid/betaine/panthenol self-recognition DES system, includes the following steps:
0.01 mol of azelaic acid, 0.01 mol of betaine, and 0.02 mol of D-panthenol were mixed uniformly, and then a resulting mixture was placed in a container, and stirred and heated at 500 rpm and 95°C for 5 hours in a natural air atmosphere to obtain a transparent and homogeneous liquid azelaic acid/betaine/panthenol self-recognition DES system;
a mass fraction of azelaic acid in the azelaic acid/betaine/panthenol self-recognition DES system was 26%;
the azelaic acid/betaine/panthenol self-recognition DES system was diluted by adding water until a mass concentration of azelaic acid was 5%, and the diluted system exhibited no phase separation or solid precipitation, that is, the DES system remained intact; and
both the azelaic acid/betaine/panthenol self-recognition DES system and the diluted system were subjected to a high-low temperature cycle test (cycle storage at - 5°C and 40°C for 15 times), and finally both the systems maintained a stable and homogeneous liquid state without solid precipitation.

After storage of the azelaic acid/betaine/panthenol self-recognition DES system at room temperature for 24 hours, a sample taken was characterized with an infrared spectrometer and thermogravimetric analysis (TGA), with test results as follows:

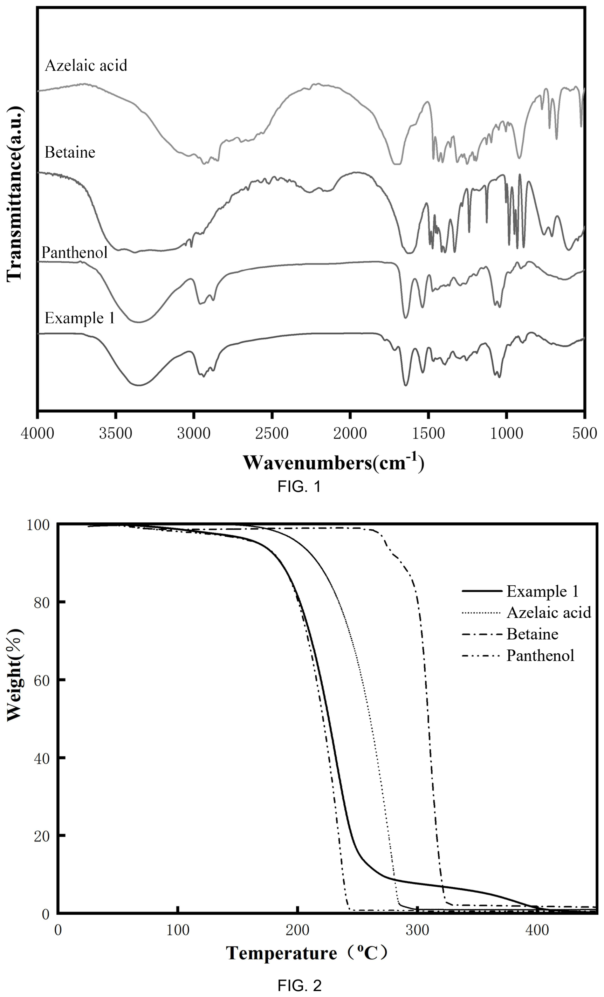

FIG. 1 shows test results of an infrared spectrum. It can be seen from FIG. 1 that an absorption peak in a waveband range of 3,500-3,200 cm⁻¹ corresponds to an absorption peak of hydroxyl groups (hydrogen bonds), and a peak of Example 1 in the waveband range shifts toward a lower wavenumber. A peak at 1,650-1,700 cm⁻¹ corresponds to a stretching vibration of carbonyl groups, and compared with three types of individual molecules, the liquid DES system in Example 1 derives a small peak at 1,710 cm⁻¹ and shows a tendency to shift toward a higher wavenumber, because the formation of hydrogen bonds by carbonyl oxygen in the system results in changes in an electron cloud density. These results indicate that hydrogen bonds are formed in the DES system.
FIG. 2 shows a dynamic TGA curve. It can be seen from FIG. 2 that the azelaic acid/betaine/panthenol self-recognition DES system exhibits a moderate weight loss temperature and an overall slower weight loss trend compared with monomer molecules. This also indicates to a certain extent that a new organic unity is formed by azelaic acid, betaine, and panthenol.

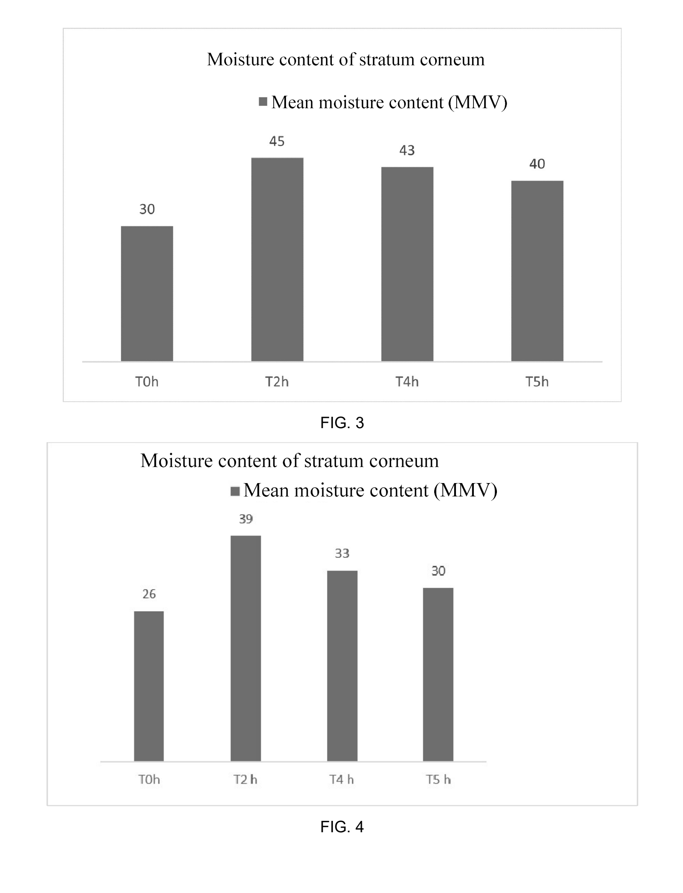

Results of moisturizing performance evaluation after diluting the azelaic acid/betaine/panthenol self-recognition DES system by adding water until a mass fraction of azelaic acid is 4% are shown in FIG. 3, and it can be seen from FIG. 3 that moisture content of human epidermis increases significantly and moisture is not lost for a long time, indicating that the system has good moisturizing performance.

### Example 2

A method for preparing an azelaic acid/betaine/panthenol self-recognition DES system, includes the following steps:
0.01 mol of azelaic acid, 0.02 mol of betaine, and 0.05 mol of DL-panthenol were mixed uniformly, and then a resulting mixture was placed in a container, and stirred and heated at 450 rpm and 90°C for 4 hours in a natural air atmosphere to obtain a transparent and homogeneous liquid azelaic acid/betaine/panthenol self-recognition DES system;
a mass fraction of azelaic acid in the azelaic acid/betaine/panthenol self-recognition DES system was 13%;
the azelaic acid/betaine/panthenol self-recognition DES system was diluted by adding water until a mass concentration of azelaic acid was 1%, and the diluted system exhibited no phase separation or solid precipitation, that is, the DES system remained intact; and
both the azelaic acid/betaine/panthenol self-recognition DES system and the diluted system were subjected to a high-low temperature cycle test (cycle storage at - 5°C and 40°C for 15 times), and finally both the systems maintained a stable and homogeneous liquid state without solid precipitation.

Results of moisturizing performance evaluation after diluting the azelaic acid/betaine/panthenol self-recognition DES system by adding water until a mass fraction of azelaic acid is 4% are shown as follows:
Mean moisture content at 0 hour was 28, mean moisture content at 2 hours was 47, mean moisture content at 4 hours was 45, and mean moisture content at 5 hours was 42.

### Example 3

A method for preparing an azelaic acid/betaine/panthenol self-recognition DES system, includes the following steps:
0.01 mol of azelaic acid, 0.015 mol of betaine, and 0.04 mol of DL-panthenol were mixed uniformly, and then a resulting mixture was placed in a container, and stirred and heated at 600 rpm and 105°C for 3 hours in a natural air atmosphere to obtain a transparent and homogeneous liquid azelaic acid/betaine/panthenol self-recognition DES system;
a mass fraction of azelaic acid in the azelaic acid/betaine/panthenol self-recognition DES system was 16%;
the azelaic acid/betaine/panthenol self-recognition DES system was diluted by adding water until a mass concentration of azelaic acid was 10%, and the diluted system exhibited no phase separation or solid precipitation, that is, the DES system remained intact; and
both the azelaic acid/betaine/panthenol self-recognition DES system and the diluted system were subjected to a high-low temperature cycle test (cycle storage at - 5°C and 40°C for 15 times), and finally both the systems maintained a stable and homogeneous liquid state without solid precipitation.

Results of moisturizing performance evaluation after diluting the azelaic acid/betaine/panthenol self-recognition DES system by adding water until a mass fraction of azelaic acid is 4% are shown as follows:
Mean moisture content at 0 hour was 31, mean moisture content at 2 hours was 49, mean moisture content at 4 hours was 46, and mean moisture content at 5 hours was 44.

### Example 4

A method for preparing an azelaic acid/betaine/panthenol self-recognition DES system, includes the following steps:
0.01 mol of azelaic acid, 0.01 mol of betaine, and 0.02 mol of D-panthenol were mixed uniformly, and then a resulting mixture was placed in a container, and stirred and heated at 500 rpm and 50°C for 1 hour in a natural air atmosphere to obtain a transparent and homogeneous liquid azelaic acid/betaine/panthenol self-recognition DES system;
a mass fraction of azelaic acid in the azelaic acid/betaine/panthenol self-recognition DES system was 26%;
the azelaic acid/betaine/panthenol self-recognition DES system was diluted by adding water until a mass concentration of azelaic acid was 5%, and the diluted system exhibited no phase separation or solid precipitation, that is, the DES system remained intact; and
both the azelaic acid/betaine/panthenol self-recognition DES system and the diluted system were subjected to a high-low temperature cycle test (cycle storage at - 5°C and 40°C for 15 times), and finally both the systems maintained a stable and homogeneous liquid state without solid precipitation.

### Example 5

A method for preparing an azelaic acid/betaine/panthenol self-recognition DES system, includes the following steps:
0.01 mol of azelaic acid, 0.01 mol of betaine, and 0.02 mol of D-panthenol were mixed uniformly, and then a resulting mixture was placed in a container, and stirred and heated at 500 rpm and 60°C for 1 hour in a natural air atmosphere to obtain a transparent and homogeneous liquid azelaic acid/betaine/panthenol self-recognition DES system;
a mass fraction of azelaic acid in the azelaic acid/betaine/panthenol self-recognition DES system was 26%;
the azelaic acid/betaine/panthenol self-recognition DES system was diluted by adding water until a mass concentration of azelaic acid was 5%, and the diluted system exhibited no phase separation or solid precipitation, that is, the DES system remained intact; and
both the azelaic acid/betaine/panthenol self-recognition DES system and the diluted system were subjected to a high-low temperature cycle test (cycle storage at - 5°C and 40°C for 15 times), and finally both the systems maintained a stable and homogeneous liquid state without solid precipitation.

### Comparative Example 1

D-panthenol was omitted on the basis of Example 1, and other conditions of Example 1 remained unchanged to obtain a product.

The obtained product was subjected to a performance test, with test results as follows:
Compared with Example 1, **when only betaine was selected as a single compatible component** for an attempt in a self-recognition DES system, a homogeneous, transparent, and stable liquid system was not obtained, and a system obtained after storage at room temperature for 24 hours was a solid-liquid mixture system. When the solid-liquid mixture system after storage at room temperature for 24 hours was diluted by adding water until a mass concentration of azelaic acid was 5%, a homogeneous and transparent aqueous solution was not obtained, but solid precipitation was observed.

### Comparative Example 2

Betaine was omitted on the basis of Example 1, and other conditions of Example 1 remained unchanged to obtain a product.

The obtained product was subjected to a performance test, with test results as follows:
Compared with Example 1, when **only panthenol was selected as a single compatible component for an attempt in a self-recognition DES system,** a homogeneous, transparent, and stable liquid DES system could be obtained, and when the liquid DES system in this comparative example was diluted by adding water until a mass concentration of azelaic acid was 5%, a homogeneous and transparent aqueous solution was obtained without solid precipitation.

Additionally, results of moisturizing performance evaluation of the system product in this comparative example are shown in FIG. 4:
It can be seen from FIG. 4 that moisture content of human epidermis increases to a certain extent, but compared with the system of Example 1, the system mentioned herein exhibits a low magnitude of moisture content increase, and a faster moisture loss rate during long-term moisturization.

### Comparative Example 3

A usage amount of betaine in Example 1 was adjusted to 0.07 mol, and other conditions of Example 1 remained unchanged to obtain a product.

The obtained product was subjected to a performance test, with test results as follows:
Compared with Example 1, **when only a feeding amount of betaine was increased,** a homogeneous, transparent, and stable liquid self-recognition system was not obtained, and a system obtained after storage at room temperature for 24 hours was a solid-liquid mixture system. When the solid-liquid mixture system after storage at room temperature for 24 hours was diluted by adding water until a mass concentration of azelaic acid was 5%, a homogeneous and transparent aqueous solution was not obtained, but solid precipitation was observed.

### Comparative Example 4

A usage amount of azelaic acid in Example 1 was adjusted to 0.03 mol, and other conditions of Example 1 remained unchanged to obtain a product.

The obtained product was subjected to a performance test, with test results as follows:
Compared with Example 1, **when only a feeding amount of azelaic acid was increased,** a homogeneous, transparent, and stable liquid self-recognition system was not obtained, and a system obtained after storage at room temperature for 24 hours was a solid-liquid mixture system. When the solid-liquid mixture system after storage at room temperature for 24 hours was diluted by adding water until a mass concentration of azelaic acid was 5%, a homogeneous and transparent aqueous solution was not obtained, but solid precipitation was observed.

### Comparative Example 5

The heating temperature in Example 1 was adjusted to 45°C, and other conditions of Example 1 remained unchanged to obtain a product.

The obtained product was subjected to a performance test, with test results as follows:
Compared with Example 1, **when only the heating temperature was lowered,** a homogeneous, transparent, and stable liquid self-recognition system was not obtained, and a system obtained after storage at room temperature for 24 hours was a solid-liquid mixture system. When the solid-liquid mixture system after storage at room temperature for 24 hours was diluted by adding water until a mass concentration of azelaic acid was 5%, a homogeneous and transparent aqueous solution was not obtained, but solid precipitation was observed.

### Comparative Example 6

The heating temperature in Example 1 was adjusted to 120°C, and other conditions of Example 1 remained unchanged to obtain a product.

The obtained product was subjected to a performance test, with test results as follows:
Compared with Example 1, **when only the heating temperature was increased,** but the system exhibited significantly deepened coloration and a relatively pungent odor, maybe due to occurrence of additional chemical reactions during a reaction process or decomposition of components in the system.

### Comparative Example 7

A heating duration in Example 1 was adjusted to 40 minutes, and other conditions of Example 1 remained unchanged to obtain a product.

The obtained product was subjected to a performance test, with test results as follows:
Compared with Example 1, **when only the heating duration was shortened,** a homogeneous, transparent, and stable liquid self-recognition system was not obtained, and a system obtained after storage at room temperature for 24 hours was a solid-liquid mixture system. When the solid-liquid mixture system after storage at room temperature for 24 hours was diluted by adding water until a mass concentration of azelaic acid was 5%, a homogeneous and transparent aqueous solution was not obtained, but solid precipitation was observed.

### Comparative Example 8

Betaine in Example 1 was replaced with theanine, and other conditions of Example 1 remained unchanged to obtain a product.

The obtained product was subjected to a performance test, with test results as follows:
Compared with Example 1, **when only one of the compatible components of the system was changed and theanine was selected to replace betaine as an attempt,** a homogeneous, transparent, and stable liquid self-recognition system was not obtained, and a system obtained after storage at room temperature for 24 hours was a solid-liquid mixture system. When the solid-liquid mixture system after storage at room temperature for 24 hours was diluted by adding water until a mass concentration of azelaic acid was 5%, a homogeneous and transparent aqueous solution was not obtained, but solid precipitation was observed.

### Comparative Example 9

Betaine in Example 1 was replaced with lauryl betaine, and other conditions of Example 1 remained unchanged to obtain a product.

The obtained product was subjected to a performance test, with test results as follows:
Compared with Example 1, **when only one of the compatible components of the system was changed and lauryl betaine was selected to replace betaine as an attempt,** a homogeneous, transparent, and stable liquid self-recognition system was not obtained, and a system obtained after storage at room temperature for 24 hours was a solid-liquid mixture system. When the solid-liquid mixture system after storage at room temperature for 24 hours was diluted by adding water until a mass concentration of azelaic acid was 5%, a homogeneous and transparent aqueous solution was not obtained, but solid precipitation was observed.

### Comparative Example 10

Betaine in Example 1 was replaced with sorbitol, and other conditions of Example 1 remained unchanged to obtain a product.

The obtained product was subjected to a performance test, with test results as follows:
Compared with Example 1, **when only one of the compatible components of the system was changed and sorbitol was selected to replace betaine as an attempt,** a homogeneous, transparent, and stable liquid self-recognition system was not obtained, and a system obtained after storage at room temperature for 24 hours was a solid-liquid mixture system. When the solid-liquid mixture system after storage at room temperature for 24 hours was diluted by adding water until a mass concentration of azelaic acid was 5%, a homogeneous and transparent aqueous solution was not obtained, but solid precipitation was observed.

### Comparative Example 11

D-panthenol in Example 1 was replaced with glycerol, and other conditions of Example 1 remained unchanged to obtain a product.

The obtained product was subjected to a performance test, with test results as follows:
Compared with Example 1, **when only one of the compatible components of the system was changed and glycerol was selected to replace panthenol as an attempt,** a homogeneous, transparent, and stable liquid self-recognition system was not obtained, and a system obtained after storage at room temperature for 24 hours was a solid-liquid mixture system. When the solid-liquid mixture system after storage at room temperature for 24 hours was diluted by adding water until a mass concentration of azelaic acid was 5%, a homogeneous and transparent aqueous solution was not obtained, but solid precipitation was observed.

### Comparative Example 12

D-panthenol in Example 1 was replaced with a mixture of polyethylene glycol 400 and propylene glycol (a mass ratio of 3:1), and other conditions of Example 1 remained unchanged to obtain a product.

The obtained product was subjected to a performance test, with test results as follows:
Compared with Example 1, when only one of the compatible components of the system was changed and the mixture of polyethylene glycol 400 and propylene glycol was selected to replace panthenol as an attempt, a homogeneous, transparent, and stable liquid self-recognition system was not obtained, and a system obtained after storage at room temperature for 24 hours was a solid-liquid mixture system. When the solid-liquid mixture system after storage at room temperature for 24 hours was diluted by adding water until a mass concentration of azelaic acid was 5%, a homogeneous and transparent aqueous solution was not obtained, but solid precipitation was observed.

### Comparative Example 13

D-panthenol in Example 1 was replaced with propylene glycol or polyethylene glycol 400, and other conditions of Example 1 remained unchanged to obtain a product.

The obtained product was subjected to a performance test, with test results as follows:
Compared with Example 1, when only one of the compatible components of the system was changed and propylene glycol or polyethylene glycol 400 was selected to replace panthenol as an attempt, a homogeneous, transparent, and stable liquid self-recognition system was not obtained, and a system obtained after storage at room temperature for 24 hours was a solid-liquid mixture system. When the solid-liquid mixture system after storage at room temperature for 24 hours was diluted by adding water until a mass concentration of azelaic acid was 5%, a homogeneous and transparent aqueous solution was not obtained, but solid precipitation was observed.

### Example 6 An Application in Cosmetics

The azelaic acid/betaine/panthenol self-recognition DES system prepared in Example 1 was used to prepare an essence serum, with a specific formula shown in Table 1 below:

**Table 1 Essence Serum Formula**

| Serial No. | Raw Material | %w/w |
|---|---|---|
| 1 | Water | To 100 |
| 2 | Glycerol | 3 |
| 3 | Butylene glycol | 3 |
| 4 | Phenoxyethanol | 0.4 |
| 5 | Caprylyl glycol | 0.4 |
| 6 | *Sphingomonas* ferment extract | 0.1 |
| 7 | Sodium hyaluronate | 0.05 |
| 8 | Polyacryloyldimethyl taurate | 0.1 |
| 9 | The azelaic acid/betaine/panthenol self-recognition DES system of Example 1 | 20 |
| 10 | White willow bark extract | 2 |
| 11 | Disodium EDTA | 0.05 |
| 12 | Portulaca oleracea extract | 2 |

A preparation method is as follows:
raw materials No. 1-5 and No. 11 were added to a main vessel, dissolved uniformly, and heated to 75°C;
raw materials No. 6-8 were added to the main vessel, and stirred uniformly; and
after cooling to 45°C, raw materials No. 9, 10, and 12 were added and stirred uniformly to complete the preparation of an essence.

Results of stability test of the essence according to the GB/T 26367-2010 standard show that: The essence serum is qualified in stability; and the prepared essence has certain moisturizing and acne-removing effects.

### Comparative Example 14

According to the formula of the essence in Table 1, the azelaic acid/betaine/panthenol self-recognition DES system of Example 1 was replaced with 5.26%w/w azelaic acid, 3.28%w/w betaine, and 11.46%w/w D-panthenol, and the essence serum was prepared as shown in Table 2 below;
a preparation process is as follows:
raw materials No. 1-5 and No. 13 were added to a main vessel, dissolved uniformly, and heated to 75°C;
raw materials No. 6-8 were added to the main vessel, and stirred uniformly; and
after cooling to 45°C, raw materials No. 9-12 and No. 14 were added and stirred uniformly to complete the preparation of an essence.

Results of stability test of the essence according to the GB/T 26367-2010 standard show that the essence serum is unqualified in stability, with solid precipitation.

**Table 2 Essence Serum Formula**

| Serial No. | Raw Material | %w/w |
|---|---|---|
| 1 | Water | To 100 |
| 2 | Glycerol | 3 |
| 3 | Butylene glycol | 3 |
| 4 | Phenoxyethanol | 0.4 |
| 5 | Caprylyl glycol | 0.4 |
| 6 | *Sphingomonas* ferment extract | 0.1 |
| 7 | Sodium hyaluronate | 0.05 |
| 8 | Polyacryloyldimethyl taurate | 0.1 |
| 9 | Azelaic acid | 5.26 |
| 10 | Betaine | 3.28 |
| 11 | D-panthenol | 11.46 |
| 12 | White willow bark extract | 2 |
| 13 | Disodium EDTA | 0.05 |
| 14 | Portulaca oleracea extract | 2 |

### Example 7 An Application in Drugs

The azelaic acid/betaine/panthenol self-recognition DES system prepared in Example 1 was used to prepare a gel agent for removing pigmentation and acne, with a specific formula shown in Table 3 below;

**Table 3 Gel Agent Formula**

| Serial No. | Raw Material | %w/w |
|---|---|---|
| 1 | Water | To 100 |
| 2 | Docusate sodium | 0.05 |
| 3 | Disodium ethylenediaminetetraacetate | 0.1 |
| 4 | Glycerol | 4 |
| 5 | Poloxamer 124 | 0.2 |
| 6 | Propylene glycol | 4 |
| 7 | SIMULGEL 600PHA | 4 |
| 8 | The azelaic acid/betaine/panthenol self-recognition DES system of Example 1 | 30 |

A preparation method was as follows:
(1) Preparation of an active phase:
   Poloxamer 124 was added to water and stirred uniformly to obtain a solution, the solution was further stirred until it was dissolved and became clear, and then the azelaic acid/betaine/panthenol self-recognition DES system of Example 1 was added and homogenized with a homogenizer at 10,000 rpm for 30 minutes for later use;
(2) preparation of an aqueous phase:
   propylene glycol and glycerol were added to water to obtain a solution, the solution was dissolved and became clear, and then docusate sodium was added and stirred at 1,000 rpm to further form a clear solution for later use;
(3) mixing:
   the aqueous phase and the active phase were sequentially added to a kettle, a beaker containing the active phase was rinsed with water, and then SIMULGEL 600 PHA was added, homogenized and stirred at 2,800 rpm for 45 minutes;
(4) filling:
   a resulting mixture was manually filled into an empty plastic tube to obtain a gel agent for treating acne.

Results of stability test of the obtained gel agent show that the gel agent is qualified in stability without solid precipitation, and has certain effects of removing pigmentation and acne.

Although the present invention has been disclosed in preferred examples, but they are not intended to limit the present invention. Anyone skilled in the art can make various changes and modifications without departing from the spirit and scope of the present invention. Therefore, the scope of protection of the present invention should be defined by the claims.

## Claims

1. A method for preparing an azelaic acid/betaine/panthenol self-recognition DES system, **characterized by** comprising the following steps:
mixing azelaic acid, betaine, and panthenol according to a molar ratio of 1:0.4-6:0.7-10, and stirring and heating to obtain an azelaic acid/betaine/panthenol self-recognition DES system.

2. The method according to claim 1, **characterized in that** the stirring and heating refers to stirring and heating at 350-850 rpm and 50-115°C for 1-15 hours.

3. The method according to claim 1, **characterized in that** an atmosphere for stirring and heating is natural air.

4. The method according to claim 1, **characterized in that** cooling to room temperature is required after stirring and heating.

5. An azelaic acid/betaine/panthenol self-recognition DES system prepared by the method according to any one of claims 1-4.

6. The azelaic acid/betaine/panthenol self-recognition DES system according to claim 5, **characterized in that** maximum content of azelaic acid in the azelaic acid/betaine/panthenol self-recognition DES system reaches up to 50 wt%.

7. An application of the azelaic acid/betaine/panthenol self-recognition DES system according to claim 5 in the field of daily chemicals or drug preparation.

8. A cosmetic product, **characterized by** containing the azelaic acid/betaine/panthenol self-recognition DES system according to claim 5.

9. The cosmetic product according to claim 8, **characterized in that** an addition amount of the azelaic acid/betaine/panthenol self-recognition DES system accounts for 0.1-70 wt%.

10. The cosmetic product according to claim 8, **characterized in that** the cosmetic product is one of an essence serum, essence lotion, spray, skincare foam, emulsion, and shampoo.

11. The cosmetic product according to claim 8, **characterized by** comprising the following formula components in percentage by mass:
glycerol (2-4%), butylene glycol (2-4%), phenoxyethanol (0.3-0.5%), caprylyl glycol (0.3-0.5%), a *Sphingomonas* ferment extract (0.05-0.15%), sodium hyaluronate (0.04-0.06%), polyacryloyldimethyl taurate (0.05-0.15%), an azelaic acid/betaine/panthenol self-recognition DES system (10-30%), a white willow bark extract (1-3%), disodium ethylenediaminetetraacetate (EDTA) (0.04-0.06%), a portulaca oleracea extract (1-3%), and a balance of water.

12. The cosmetic product according to claim 8, **characterized in that** the cosmetic product is of an aqueous type or a refreshing type.

13. A drug for removing pigmentation and acne, **characterized by** containing the azelaic acid/betaine/panthenol self-recognition DES system according to claim 5.

14. The drug according to claim 13, **characterized in that** the drug is one of a gel agent, spray, liquid preparation, and cream.

15. The drug according to claim 13, **characterized in that** an addition amount of the azelaic acid/betaine/panthenol self-recognition DES system accounts for 0.1-70 wt%.

16. The drug according to claim 13, **characterized by** comprising the following formula components in percentage by mass:
an azelaic acid/betaine/panthenol self-recognition DES system (10-30%), docusate sodium (0.03-0.06%), disodium EDTA (0.05-0.15%), glycerol (3-5%), poloxamer 124 (0.1-0.3%), propylene glycol (3-5%), SIMULGEL 600 PHA (3-5%), and a balance of water.

17. A method for synergistically improving a moisturizing effect of azelaic acid by using betaine and panthenol, **characterized by** comprising the following steps: mixing azelaic acid, betaine, and panthenol according to a molar ratio of 1:0.4-6:0.7-10, and stirring and heating to obtain an azelaic acid/betaine/panthenol self-recognition DES system with good moisturizing effect.

18. A method for enhancing water solubility and stability of azelaic acid by using betaine and panthenol, **characterized by** comprising the following steps:
mixing azelaic acid, betaine, and panthenol according to a molar ratio of 1:0.4-6:0.7-10, and stirring and heating to obtain an azelaic acid/betaine/panthenol self-recognition DES system with good water solubility and stability.
